# EUROPEAN PATENT APPLICATION

(11) **EP 1 518 518 A2**
(43) Date of publication of application: **30.03.2005**
(21) Application number: 04466016.5
(22) Date of filing: 01.07.2004
(51) Int. Cl.: A61F 2/06

(54) **Stent designed to stop esophageal variceal bleeding**

(30) Priority: 25.09.2003 CZ 20032591
(71) Applicant: Volenec, Karel, 500 06 Hradec Kralové (CZ)
(72) Inventor: Danis, Jan, 2410 Hainburg an der Donau (AT); Volenec, Karel, 500 02 Hradec Kralové (CZ)
(74) Representative: Brykner, Jan (CZ)

(57) **Abstract**

The stent designed to stop esophageal variceal bleeding consists of the elastic mesh (1), the active length (that part of the stent that should cover the bleeding varices) is covered with impermeable foil (4). At least one end of the elastic mesh (1) is provided with a funnel-shaped throat (2). After being implanted, the elastic mesh (1) presses the impermeable foil (4) against the esophageal wall and thus the bleeding is prevented. After the bleeding is stopped, the stent removal is made by grasping and pulling the loop (3) of the stainless-steel elastic pursing string (5), which is passed through the eyelets (6) made of the wire at both edges of the stent. The elastic pursing string (5) enables the concentric and symmetric constriction of the stent diameter. The elastic mesh (1) with the funnel-shaped throat (2) and the impermeable foil (4) can be made of biodegradable material which produces the stent disintegration after a certain period since the bleeding is stopped.

## Description

### Technical field

The invention concerns the stent, which consists of elastic mesh, designed to stop bleeding from esophageal varices.

### Current situation in the technical field

Until now, any known stent was suitable to be used in order to stop esophageal variceal bleeding. The most frequently used method of treatment is the use of balloon catheters, which has low effect and their insertion is very slow what does not correspond to the need of a quick intervention. The other methods of stopping the variceal bleeding are variceal ligation or injection of tissue glues (emergency variceal sclerotherapy). But these methods are timeconsuming and associated with enormous loss of blood.

The goal of this invention is to create a stent suitable to be used to stop the esophageal variceal bleeding.

### Principle of invention

The stent consist of elastic mesh, which is covered with impermeable foil and at least one end of the elastic mesh is provided with funnel-shaped throat (flare). The funnel is equipped with a elastic pursing string and loop. Another option is that the mesh with the foil is made of biodegradable material. Both alternatives are possible.

If the bleeding occurs, the stent is introduced by a special designed catheter. The impermeable foil is pressed against the esophageal wall by the expansion elastic force developed by the elastic mesh and thus stops the bleeding. The funnel /funnels keeps/keep the stent in required position. Mostly loop, which is created on both ends of the metalic elasting pursing string, pulled through the eyelets that are made of the wire at both edges of the stent mesh. This system enables the symmetric concentric constriction of the stent while being pulled out. The stent removal or changing of its position is performed by grasping the loop of the pursing string. While being pulled, the string reduces the stent diameter and facilitates its easy removal. If the stent and the impermeable foil are made of biodegradable material, there is no need to remove the stent after it has stopped the bleeding because it disintegrates after certain time.

### Overview of the pictures on the drawing

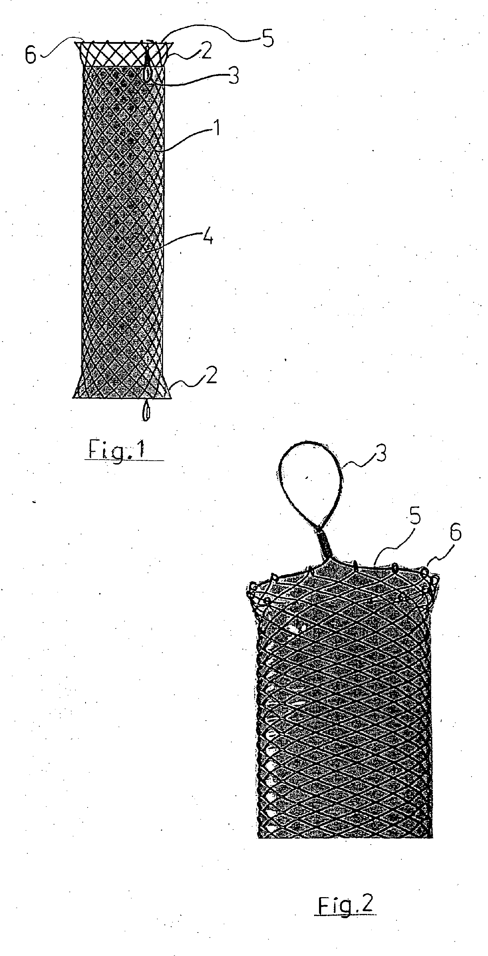

Stent, according to the invention, equipped with the pursing string is presented on the herewith attached drawing. Fig.1 presents the scheme of the entire stent, fig.2 presents the detailed view of the edge of stent with the elastic pursing string, pulled through the snares along the stent circumference.

### Example of the stent design

The stent described on the drawing is made by elastic mesh 1 and funnel-shaped throat 2 at both ends. The elastic mesh 1 is provided with the eyelets 6 formed by the wire on both edges along the stent circumference, which the elastic pursing string 5 is passed through, whose ends create the loops 3. The active length (that part of the stent that should cover the bleeding varices) and one end of the stent are covered with the impermeable foil 4.

After being implanted, the elastic mesh 1 presses the impermeable foil 4 against the esophageal wall and thus the bleeding is prevented. The funnel-shaped throat 2 at the stent ends keep the stent in the required position. The stent removal is performed by grasping and pulling the loop 3 of the stainless - steel pursing string 5 enables the concentric and symmetric constriction of the stent diameter, while its loop 3 is being grasped and pulled. This system enables easy removal of the stent.

## Claims

1. Stent designed to stop variceal bleeding, consisting of elastic mesh, **characterized in that** said elastic mesh (1), which is in the active part covered with the impermeable foil (4) and at least one end of the elastic mesh (1) is provided with a funnel-shaped throat (2).

2. The stent according to Claim 1, **characterized in that**, where present, said funnel-shaped throat (2) is provided with the loop (3).

3. The stent according to Claim 1, **characterized in that** said elastic mesh (1) with the funnel-shaped throat (2) and the impermeable foil (4) are made of biodegradable material .

4. The stent according to Claim 2, **characterized in that** said loop (3), which is created on both ends of the metallic elastic pursing string (5), pulled through the eyelets (6) that are made of the wire at both edges of the elastic mesh (1).
